# EUROPÄISCHE PATENTSCHRIFT

(11) **EP 1 024 126 B1**
(45) Veröffentlichungstag und Bekanntmachung des Hinweises auf die Patenterteilung: **29.10.2003**
(21) Anmeldenummer: 99124543.2
(22) Anmeldetag: 09.12.1999
(51) Int. Cl.: C07C 29/52, C07C 45/33, C07C 35/02, C07C 35/20, C07C 35/205, C07C 49/385, C07C 49/413

(54) **Verfahren zur Herstellung von cyclischen Alkoholen und Ketonen**
Process for the preparation of cyclic alcohols and ketones
Procédé pour la préparation d' alcools et de cétones cycliques

(30) Priorität: 27.01.1999 DE 19903152
(43) Veröffentlichungstag der Anmeldung: 02.08.2000
(73) Patentinhaber: Degussa AG, 40474 Düsseldorf (DE)
(72) Erfinder: Esser, Peter Ernst, Dr., 45657 Recklinghausen (DE); Schiffer, Thomas, Dr., 45721 Haltern (DE); Günzel, Bernd, Dr., 45721 Haltern (DE); Oenbrink, Georg, Dr., 48249 Dülmen (DE)

(56) Entgegenhaltungen:
- DE-A- 1 518 661
- FR-A- 1 577 815
- GB-A- 1 035 624
- US-A- 5 767 320

## Beschreibung

Die Erfindung betrifft ein Verfahren zur Herstellung von cyclischen Alkoholen und Ketonen mit 7 bis 16 Kohlenstoffatomen durch Oxidation von Cycloalkanen mit 7 bis 16 Kohlenstoffatomen mit Sauerstoff enthaltenden Gasen in Gegenwart von in Wasser und aliphatischen oder cycloaliphatischen Kohlenwasserstoffen schwerlöslichen Übergangsmetallkatalysatoren und Borverbindungen, insbesondere Säuren, Oxiden oder Estern des Bors.

### Stand der Technik

Im Vergleich zur Oxidation von Cyclohexan mit Sauerstoff enthaltenden Gasen reagieren größere alicyclische Kohlenwasserstoffe nur langsam zu den entsprechenden Alkoholen (OL) und Ketonen (ON). Um die Reaktion an Cycloalkanen mit 7 bis 16 Kohlenstoffatomen zu beschleunigen, können Borsäure und Borsäurederivate und/oder Übergangsmetallkatalysatoren in Form ihrer löslichen Salze verwendet werden.

Gegenüber der nicht katalysierten Reaktion bewirkt die Borsäure eine Erhöhung der Reaktionsgeschwindigkeit und der Umsatzrate wie in DRP 552 886, in DE 16 43 825 sowie in den Artikeln von H. Grasemann et al. in Erdöl, Kohle, Erdgas und Petrochemie 18 (1965), 360 und 22 (1969), 751 beschrieben. Die Borsäure bewirkt, daß sich der Anteil an Keton im Produktgemisch auf ein Verhältnis verringert, welches typischerweise bei 1 : 8 oder weniger liegt (NL 6 505 838).

Um den Anteil an Keton im Produktgemisch zu erhöhen, können Übergangsmetallkatalysatoren verwendet werden. In US 4 341 907 wird die Benutzung von Cobalt-2-ethylhexanoat als Katalysator bei Reaktionstemperaturen von 130 bis 180 °C bei einer Cobaltkonzentration von mindestens 1000 ppm beschrieben. Die Reaktion wird in Anwesenheit von Pyridin durchgeführt. Dies erfordert bei einer Aufarbeitung eine zusätzliche Trennstufe. Pyridin besitzt einen charakteristischen, unangenehmen Geruch. Zudem gibt es in der Literatur mehrere Hinweise, daß Pyridin im Verdacht steht, carcinogen zu sein.

Nach DE 11 11 177 erreicht man bei 142 °C einen Umsatz von ungefähr 10 % und ein ON/OL-Verhältnis von ca. 1 : 1 bei einer Selektivität von ca. 50 %. Aufgrund der geringen Selektivität fallen neben dem gewünschten ON/OL-Gemisch durch Überoxidation große Mengen eines öligen Rückstandes an, der entsorgt oder aufgearbeitet werden muß. Die Aufarbeitung erfordert einen erheblichen Aufwand und führt nur teilweise zu Dodecandisäure als verwertbarem Produkt.

In DE 22 23 327 wird der Einsatz von Cobaltnaphthenat als Katalysator zur Oxidation von Cyclohexan beschrieben. Bei 160 °C läßt sich auf diese Weise ein Umsatz von 6,5 % erreichen. Die Oxidation von Cyclododecan wird jedoch ohne Cobaltsalze, dafür mit Hilfe von Borsäure oder ähnlichen Borverbindungen beschrieben.

In DE 16 18 077 wird die Oxidation von Cycloalkanen in Gegenwart "üblicher Oxidationskatalysatoren" und in Anwesenheit von 0,05 % bis 3 % alkylsubstituierter aromatischer Kohlenwasserstoffe beansprucht. Als Katalysatoren werden konkret Cobaltnaphthenat und andere lösliche Cobaltsalze benannt. Alle angegebenen Beispiele behandeln ausschließlich die Oxidation von Cyclohexan, wobei das ON/OL-Gemisch mit einer Selektivität von 76-80 % und einem Cyclohexanumsatz von etwa 3,5 % anfällt.

Nach GB 14 28 964 wird ein Cobaltoximderivat als Katalysator zur Oxidation von Cyclododecan bei 165 °C und Normaldruck verwendet. Man erhält auf diese Weise 8,5 % eines ON/OL-Gemisches bezogen auf eingesetztes Cyclododecan.

Die Kombination von Borsäure und Co/Mn-Salzen wird in US 3 419 615 im Beispiel 5 erwähnt. Unterhalb von 155 °C findet kaum Umsetzung statt. Erst bei Temperaturen von 160 bis 170 °C wird ein Umsatz von etwa 25 % bei diskontinuierlicher und etwa 15 % bei kontinuierlicher Reaktionsführung erreicht. Oberhalb von 170°C finden unerwünschte Zersetzungen statt. Dabei werden 0,1 % bis 10 Mol-% Schwermetallsalz bezogen auf die Gesamtmenge eingesetzt.

Bei der Aufarbeitung der Reaktionsmischung wird die organische Phase üblicherweise mit Wasser oder Alkalilaugen gewaschen. Die oben erwähnten Verfahren verwenden gut wasserlösliche Schwermetallverbindungen, die bei der Aufarbeitung in die wäßrige Phase überführt werden. Eine Rückgewinnung der Schwermetalle aus der wäßrigen Phase ist aufwendig. Andererseits schränkt die hohe Schwermetallfracht im Abwasser einen großtechnischen Einsatz dieser Verfahren ein.

In US 5 767 320 wird die Oxidation von Cyclohexan in Gegenwart von Phthalocyaninkomplexen verschiedener Schwermetalle als alleinigem Katalysator beschrieben. Bei 20 bis 80 °C findet jedoch keine Oxidation höhergliedriger Cycloverbindungen statt.

In FR-A-1 577 815 wird erwähnt, dass man die Metalle in Form von mineralischen Salzen, die auf Trägermaterialien aufgezogen werden, einsetzen kann. Diese geträgerten Materialien haben aber die Eigenschaft, dass die Salze in Wasser alle eine mehr oder weniger hohe Löslichkeit aufweisen, so dass sie beim Aufarbeiten der Reaktionsmischung mit Wasser vom Trägermaterial abgelöst werden und in löslicher Form in das Abwasser gelangen. Damit ist gemäß FR-A-1 577 815 eine Wiederverwendung des Katalysators nicht mehr möglich.

### Gegenstand der Erfindung

Es wurde nun gefunden, daß bei Einsatz von in Wasser und aliphatischen oder cycloaliphatischen Kohlenwasserstoffen schwerlöslichen Übergangsmetallkatalysatoren (schwerlösliche Komplexe, Komplexsalze oder Salze von Übergangsmetallen), insbesondere Phthalocyaninkomplexe oder substituierte Phthalocyaninkomplexe von Metallen der 6. bis 12. Gruppe, in Gegenwart von Borverbindungen, insbesondere Säuren, Oxiden oder Estern des Bors, wesentliche Verfahrensverbesserungen bei der Oxidation größerer Cycloalkane erzielt werden können.

Gegenstand der Erfindung ist mithin ein Verfahren zur Herstellung von cyclischen Alkoholen und Ketonen mit 7 bis 16, insbesondere 8 bis 12 Kohlenstoffatomen durch Oxidation von Cycloalkanen mit 7 bis 16, insbesondere 8 bis 12 Kohlenstoffatomen mit Sauerstoff enthaltenden Gasen in Gegenwart von in Wasser und aliphatischen oder cycloaliphatischen Kohlenwasserstoffen schwerlöslichen Übergangsmetallkatalysatoren mit Metallen der 6. bis 12. Grupppe in Gegenwart von Borverbindungen, insbesondere Säuren, Oxiden oder Estern des Bors, wobei die schwerlöslichen Übergangsmetallkatalysatoren nach Beendigung der Reaktion durch ein mechanisches Trennverfahren abgetrennt werden.

Als Verfahrensverbesserungen sind insbesondere
- niedrigere Reaktionstemperaturen, insbesondere Temperaturen unter 150 °C
- verbesserte Umsatzraten
- erhöhter Anteil an cyclischen Ketonen im Gemisch und
- leichtere Aufarbeitbarkeit des Reaktionsgemisches
zu nennen.

Die in dem erfindungsgemäßen Verfahren verwendeten Übergangsmetallkatalysatoren (Übergangsmetallkomplexe, Übergangsmetallkomplexsalze oder Übergangsmetallsalze) sind in der als Solvens verwendeten organischen Phase und insbesondere in der zur Aufarbeitung der Reaktionsmischung verwendeten wäßrigen Phase schwer oder sehr schwer löslich.

Die geringe Löslichkeit der erfindungsgemäß eingesetzten Übergangsmetallkatalysatoren hat zur Folge, daß diese nahezu vollständig heterogen als fein verteilter Feststoff (Suspension) in der Reaktionsmischung vorliegen. Bei guten Umsatzraten ergeben sich dadurch insbesondere deutliche Vorteile bei der Aufarbeitung und Abtrennung der Übergangsmetalle, da sie sich durch mechanische Trennverfahren wie Filtration, Sedimentation oder Zentrifugieren leicht abtrennen lassen.
Durch die sehr geringe Wasserlöslichkeit der erfindungsgemäß eingesetzten Übergangsmetallkomplexe, Übergangsmetallkomplexsalze oder Übergangsmetallsalze gelangt der Katalysator bei der Aufarbeitung der Reaktionsmischung nur noch in Spuren in die wäßrige Phase. Somit läßt sich die Metallfracht im Abwasser im Vergleich zum Stand der Technik erheblich reduzieren.

Der aus der Reaktionsmischung isolierte Katalysator kann getrocknet und danach erneut in der Synthese eingesetzt werden.

Als Übergangsmetalle können in den erfindungsgemäß eingesetzten Komplexen, Komplexsalzen oder Salzen insbesondere Eisen, Cobalt, Chrom, Kupfer und Mangan sowie deren Mischungen eingesetzt werden. Vorzugsweise werden Cobalt oder Mangan als Übergangsmetall eingesetzt.

Als Komplexe sind insbesondere die Komplexe der Phthalocyanine und der substituierten Phthalocyanine geeignet, wobei bei letzteren die 1 bis 16 Substituenten die Positionen 1, 2, 3, 4, 8, 9, 10, 11, 15, 16, 17, 18, 22, 23, 24, 25 im Phthalocyaningerüst einnehmen können. Als Substituenten werden elektronenziehende Gruppen bevorzugt, wobei gegebenenfalls auch verschiedene Substituenten in einem Molekül vorkommen können. Als Beispiele für elektronenziehende Gruppen seien Halogen-, Nitro- und Cyanogruppen genannt.

Die Konzentration des Metalles des Übergangsmetallkatalysators beträgt bezogen auf die Gesamtmischung 0,0005 bis 1 Gew.-%, vorzugsweise 0,003 bis 1 Gew.-%.

Als Borverbindungen sind insbesondere die Säuren des Bors (ortho- und meta-Borsäure), Boroxid und Ester des Bors wie Borsäuretrimethyl- oder -triethylester, geeignet. Vorzugsweise werden ortho- und meta-Borsäure verwendet.

Die Konzentration der Borverbindung, insbesondere also der Borsäure beträgt 0,1 bis 25 Gew.-%, insbesondere 0,1 bis 10 Gew.-% bezogen auf die Gesamtmischung.

Als Solvens werden apolar aprotische Lösungsmittel wie zum Beispiel Alkane oder Cycloalkane bevorzugt. Besonders vorteilhaft ist der Einsatz des jeweiligen Eduktes als Solvens.

Die Oxidation wird im Temperaturbereich von 90 bis 160 °C, insbesondere bei 130 bis 150 °C und besonders bevorzugt bei 140 bis 150 °C durchgeführt.

Die Reaktion wird üblicherweise bei Normaldruck durchgeführt. Es kann aber auch ein Überdruck bis etwa 10 bar, vorzugsweise bis 5 bar eingestellt werden.

Die Reaktion kann diskontinuierlich oder kontinuierlich durchgeführt werden.

Als Oxidationsgas kann im einfachsten Fall (getrocknete) Luft eingeleitet werden. Es ist aber auch möglich, durch Einleiten von Stickstoff und/oder Luft und/oder Sauerstoff gewünschte Sauerstoff-Konzentrationen im Eingangs- oder Abgas gezielt einzustellen.

### Beispiele:

Die folgenden Beispiele sollen die Erfindung näher erläutern.

### Beispiele zu den Oxidationsversuchen

### Allgemeines

Alle Versuche wurden in einem 5 l Autoklav aus rostfreiem V4A-Stahl durchgeführt. Stickstoff und Sauerstoff wurden oberhalb des Rührwerkes direkt in die flüssige Phase eingeleitet. Alle Umsetzungen erfolgten isotherm. Dabei wurde die Reaktionstemperatur durch eine externe Beheizung über die Reaktorwand und einen innen liegenden Kühlkreislauf zur Abführung der Reaktionswärme geregelt. Der Kühlkreislauf bewirkte zudem ein schnelles Abkühlen der Produkte nach Beendigung der Reaktion. Das Abgas verließ den Reaktor durch einen zweistufigen Metallkühler und wurde in einer nachgeschalteten Gaswäsche von mitgerissenen Produkten befreit.

### Vergleichsbeispiel V1

Der Reaktor wurde mit 3,670 kg flüssigem CDAN (Cyclododecan) und 45 g ortho-Borsäure gefüllt. Die Mischung wurde unter Stickstoffbegasung aufgeheizt. Bei einer Temperatur von 145 °C wurde so viel Sauerstoff zudosiert, daß der Sauerstoffgehalt im Abgas einen Wert von 8 Vol-% nicht überschritt. Nach dem Anspringen der Reaktion wurde eine Menge von 120 g ortho-Borsäure über einen Feststoffsilo in den Reaktor dosiert. Innerhalb von 2,5 Stunden wurde dieser Vorgang zweimal mit je 65 g Borsäure wiederholt. Nach einer Reaktionszeit von 5,0 Stunden wurde die Mischung abgekühlt und der Reaktoraustrag mit Wasser hydrolysiert. Die organische Phase wurde von der wäßrigen Borsäure abgetrennt und gaschromatographisch untersucht. Es konnte ein CDAN-Umsatz von 13% und Ausbeuten für CDOL, bzw. CDON von 10,3% und 0,7% gemessen werden. Dies entspricht einer Gesamtselektivität von 84,6% bei einem OL/ON-Verhältnis von 14,5.

### Vergleichsbeispiel V2

3,62 kg CDAN und 50 g ortho-Borsäure wurden mit zusätzlich 32 g Cobaltacetat-Tetrahydrat bei 145 °C in der in Beispiel 1 beschriebenen Weise mit sauerstoffhaltigen Gasen und weiteren 250 g ortho-Borsäure umgesetzt. Nach einer Reaktionszeit von 4,3 Stunden konnte ein CDAN-Umsatz von 22,5% und Ausbeuten von 16,0% CDOL und 2,3% CDON bei einem OL/ON-Verhältnis von 6,9 gemessen werden. Dies entspricht einer Gesamtselektivität von 81,7%.

### Vergleichsbeispiel V3

Analog zu Vergleichsbeispiel V2 wurden 3,76 kg CDAN, 48 g ortho-Borsäure und 46 g Cobaltnaphthenat bei 145 °C mit sauerstoffhaltigen Gasen und weiteren 250 g ortho-Borsäure umgesetzt. Nach einer Reaktionszeit von 5,1 Stunden konnte ein CDAN-Umsatz von 21,0% und Ausbeuten von 15,8% CDOL und 2,0% CDON bei einem OL/ON-Verhältnis von 7,8 gemessen werden. Dies entspricht einer Gesamtselektivität von 84,5%.

### Beispiel 1

Analog zu Vergleichsbeispiel V2 wurden 3,642 kg CDAN, 47 g ortho-Borsäure und 5,0 g Cobaltphthalocyanin bei 145 °C mit sauerstoffhaltigen Gasen und weiteren 250 g ortho-Borsäure umgesetzt. Nach einer Reaktionszeit von 4,5 Stunden konnte ein CDAN-Umsatz von 19,6% und Ausbeuten von 13,0% CDOL und 3,6% CDON bei einem OL/ON-Verhältnis von 3,6 gemessen werden. Dies entspricht einer Gesamtselektivität von 84,7%.

### Beispiel 2

Analog zu Vergleichsbeispiel V2 wurden 3,475 kg CDAN, 47 g ortho-Borsäure und 12,2 g Kupferphthalocyanin bei 145 °C mit sauerstoffhaltigen Gasen und weiteren 250 g ortho-Borsäure umgesetzt. Nach einer Reaktionszeit von 4,9 Stunden konnte ein CDAN-Umsatz von 20,6% und Ausbeuten von 14,4% CDOL und 3,2% CDON bei einem OL/ON-Verhältnis von 4,5 gemessen werden. Dies entspricht einer Gesamtselektivität von 85,4%.

### Beispiel 3

Analog zu Vergleichsbeispiel V2 wurden 3,637 kg CDAN, 48 g ortho-Borsäure und 6,0 g Kupferphthalocyanin bei 145 °C mit sauerstoffhaltigen Gasen und weiteren 250 g ortho-Borsäure umgesetzt. Nach einer Reaktionszeit von 5,8 Stunden konnte ein CDAN-Umsatz von 22,1% und Ausbeuten von 15,8% CDOL und 3,1% CDON bei einem OL/ON-Verhältnis von 5,0 gemessen werden. Dies entspricht einer Gesamtselektivität von 85,5%.

### Beispiel 4

Analog zu Vergleichsbeispiel V2 wurden 3,538 kg CDAN, 48 g ortho-Borsäure und 12,2 g Manganphthalocyanin bei 145 °C mit sauerstoffhaltigen Gasen und weiteren 250 g ortho-Borsäure umgesetzt. Nach einer Reaktionszeit von 4,8 Stunden konnte ein CDAN-Umsatz von 18,6% und Ausbeuten von 13,4% CDOL und 2,5% CDON bei einem OL/ON-Verhältnis von 5,3 gemessen werden. Dies entspricht einer Gesamtselektivität von 85,5%.

### Beispiel 5

Analog zu Vergleichsbeispiel V2 wurden 3,506 kg CDAN, 50 g ortho-Borsäure und 2,0 g Cobalthexadecafluorophthalocyanin bei 145 °C mit sauerstoffhaltigen Gasen und weiteren 250 g ortho-Borsäure umgesetzt. Nach einer Reaktionszeit von 6,1 Stunden konnte ein CDAN-Umsatz von 28,8% und Ausbeuten von 20,4% CDOL und 3,2% CDON bei einem OL/ON-Verhältnis von 6,4 gemessen werden. Dies entspricht einer Gesamtselektivität von 82,3%.

### Beispiel 6

Analog zu Vergleichsbeispiel V2 wurden 3,402 kg CDAN, 47 g ortho-Borsäure und 3 g Kupferhexadecafluorophthalocyanin bei 145 °C mit sauerstoffhaltigen Gasen und weiteren 250 g ortho-Borsäure umgesetzt. Nach einer Reaktionszeit von 5,6 Stunden konnte ein CDAN-Umsatz von 25,0% und Ausbeuten von 18,0% CDOL und 2,6% CDON bei einem OL/ON-Verhältnis von 6,9 gemessen werden. Dies entspricht einer Gesamtselektivität von 82,4%.

Die Ergebnisse der Oxidationsversuche sind in der folgenden Tabelle zusammengefaßt. Dabei zeigt sich die Verbesserung des Umsatzes und des CDON-Anteils im Produktgemisch durch Cobaltphthalocyanin besonders deutlich.

| **Beispiel** | **Katalysator** | **Metall- Gehalt** **(ppm)** | **Umsatz** **(%)** | **Selektivität** | **OL/ON** | **Reaktions- zeit (h)** |
|---|---|---|---|---|---|---|
| V 1 | - | - | 13,0 | 84,6 | 14,5 | 5 |
| V 2 | Co(AcO)₂ | 1850 | 22,5 | 81,7 | 6,9 | 4,3 |
| V 3 | Co-Naphthenat | 910 | 21,0 | 84,5 | 7,8 | 5,1 |
| 1 | Co-Phthalocyanin | 130 | 19,6 | 84,7 | 3,6 | 4,5 |
| 2 | Cu-Phthalocyanin | 319 | 20,6 | 85,4 | 4,5 | 4,9 |
| 3 | Cu-Phthalocyanin | 157 | 22,1 | 85,5 | 5,0 | 5,8 |
| 4 | Mn-Phthalocyanin | 115 | 18,6 | 85,5 | 5,3 | 4,8 |
| 5 | Co-F₁₆-Phthalocyanin | 31 | 28,8 | 82,3 | 6,4 | 6,1 |
| 6 | Cu-F₁₆-Phthalocyanin | 49 | 25,0 | 82,4 | 6,9 | 5,6 |

### (Die Angaben über die Metallgehalte beinhalten die von den Herstellern angegebenen Reinheiten der Phthalocyanine.)

### Beispiele zur Aufarbeitung der Reaktionsmischungen

### Vergleichsbeispiel

Das Oxidat von Vergleichsbeispiel 2 wird durch die Zugabe von Wasser bei 90 °C hydrolysiert. Die organische Phase wird im Scheidetrichter abgetrennt. In der organischen Phase lassen sich nur 16 ppm Cobalt nachweisen. Das Cobaltacetat ist zu mehr als 98% in der wäßrigen Borsäurelösung gelöst und muß mit der Borsäure aufgearbeitet werden. Dabei fallen erhebliche Mengen eines mit Cobalt belasteten Abwassers an.

### Beispiel 7

Das Oxidat von Beispiel 1 wird durch Zugabe von Wasser bei 90 °C hydrolysiert. Die organische Phase wird im Scheidetrichter abgetrennt. Das in Wasser unlösliche Cobaltphthalocyanin wird durch Filtration abgetrennt und im Vakuum bei 60-80 °C getrocknet. Auf diese Weise läßt sich der Katalysator nahezu quantitativ wiedergewinnen und erneut in der Reaktion einsetzen. Die cobaltfreie, wäßrige Borsäurelösung kann der Aufarbeitung (z.B. Kristallisation) zugeführt werden.

## Patentansprüche

1. Verfahren zur Herstellung von cyclischen Alkoholen und Ketonen mit 7 bis 16 Kohlenstoffatomen,
**dadurch gekennzeichnet, dass**
Cycloalkane mit 7 bis 16 Kohlenstoffatomen mit Hilfe von Sauerstoff enthaltenden Gasen in Gegenwart von in Wasser und aliphatischen oder cycloaliphatischen Kohlenwasserstoffen schwerlöslichen Übergangsmetallkatalysatoren, welche aus Phthalocyanin und Metallen der 6. bis 12. Gruppe aufgebaut sind, in Gegenwart von Borverbindungen oxidiert werden, wobei die schwerlöslichen Übergangskatalysatoren nach Beendigung der Reaktion durch ein mechanisches Trennverfahren abgetrennt werden und wobei die Konzentration des Metalls des Übergangsmetallkatalysators 0,0005 bis 1 Gew.-% bezogen auf die Gesamtmischung beträgt.

2. Verfahren nach Anspruch 1,
**dadurch gekennzeichnet,**
**dass** in den Übergangsmetallkatalysatoren als Übergangsmetall Eisen, Cobalt, Chrom, Kupfer oder Mangan eingesetzt wird.

3. Verfahren nach Anspruch 1,
**dadurch gekennzeichnet,**
**dass** als Übergangsmetallkatalysatoren Komplexe der substituierten Phthalocyanine eingesetzt werden.

4. Verfahren nach Anspruch 3,
**dadurch gekennzeichnet,**
**dass** die substituierten Phthalocyanine elektronenziehende Substituenten enthalten.

5. Verfahren nach Anspruch 4,
**dadurch gekennzeichnet,**
**dass** die elektronenziehenden Substituenten Halogen-, Nitro- und/oder Cyanogruppen sind.

6. Verfahren nach einem der vorhergehenden Ansprüche,
**dadurch gekennzeichnet,**
**dass** als Borverbindung ortho- oder meta-Borsäure eingesetzt wird.

7. Verfahren nach einem der vorhergehenden Ansprüche,
**dadurch gekennzeichnet,**
**dass** die Konzentration der Borverbindung 0,1 bis 25 Gew.-% beträgt.

8. Verfahren nach einem der vorhergehenden Ansprüche,
**dadurch gekennzeichnet,**
**dass** die Konzentration der Borverbindung 0,1 bis 10 Gew.-% beträgt.

9. Verfahren nach einem der vorhergehenden Ansprüche,
**dadurch gekennzeichnet,**
**dass** Cycloalkane mit 8 bis 12 Kohlenstoffatomen als Ausgangsverbindungen eingesetzt werden.

10. Verfahren nach einem der vorhergehenden Ansprüche,
**dadurch gekennzeichnet,**
**dass** die Reaktionstemperatur 90 bis 160 °C beträgt.

11. Verfahren nach einem der vorhergehenden Ansprüche,
**dadurch gekennzeichnet,**
**dass** die Reaktionstemperatur 130 bis 150 °C beträgt.

## Claims

1. A process for preparing cyclic alcohols and ketones having from 7 to 16 carbons, **characterized in that** it comprises oxidizing cycloalkanes having from 7 to 16 carbons using oxygen-containing gases in the presence of sparingly water-soluble and sparingly aliphatic or cycloaliphatic hydrocarbon-soluble transition metal catalysts which are made up of phthalocyanine and metals of Group 6 to Group 12 in the presence of boron compounds, the sparingly soluble transition metal catalysts being separated off by a mechanical separation process after completion of the reaction, and the concentration of the metal of the transition metal catalyst being from 0.0005 to 1% by weight, based on the total mixture.

2. A process according to claim 1, **characterized in that**, in the transition metal catalysts, iron, cobalt, chromium, copper or manganese is used as transition metal.

3. A process according to claim 1, **characterized in that**, complexes of substituted phthalocyanines are used as transition metal catalysts.

4. A process according to claim 3, **characterized in that** the substituted phthalocyanines comprise electron-withdrawing substituents.

5. A process according to claim 4, **characterized in that** the electron-withdrawing substituents are halogen, nitro and/or cyano groups.

6. A process according to any one of the preceding claims, **characterized in that** the boron compound used is orthoboric or metaboric acid.

7. A process according to any one of the preceding claims, **characterized in that** the concentration of the boron compound is from 0.1 to 25% by weight.

8. A process according to any one of the preceding claims, **characterized in that** the concentration of the boron compound is from 0.1 to 10% by weight.

9. A process according to any one of the preceding claims, **characterized in that** cycloalkanes having from 8 to 12 carbons are used as starting compounds.

10. A process according to any one of the preceding claims, **characterized in that** the reaction temperature is from 90 to 160°C.

11. A process according to any one of the preceding claims, **characterized in that** the reaction temperature is from 130 to 150°C.

## Revendications

1. Procédé de préparation d'alcools et de cétones cycliques comportant de 7 à 16 atomes de carbone,
**caractérisé en ce que**
l'on oxyde des cycloalcanes comportant de 7 à 16 atomes de carbone avec des gaz contenant de l'oxygène en présence d'eau et de catalyseurs de métaux de transition difficilement solubles dans l'eau et dans les hydrocarbures aliphatiques ou cycloaliphatiques, les catalyseurs étant constitués de phtalocyanines et de métaux des 6e à 12e groupes de la classification périodique des éléments, en présence de composés de bore, les catalyseurs de transition difficilement soluble étant séparés après la fin de la réaction par un procédé de séparation mécanique, et la concentration du métal du catalyseur de métal de transition étant de 0,0005 à 1 % en poids par rapport à l'ensemble du mélange.

2. Procédé selon la revendication 1,
**caractérisé en ce que**
dans les catalyseurs en métaux de transition on utilise comme métal de transition le fer, le cobalt, le chrome, le cuivre ou le manganèse.

3. Procédé selon la revendication 1,
**caractérisé en ce qu'**
on utilise comme métal de transition les complexes de phtalocyanine substitués.

4. Procédé selon la revendication 3,
**caractérisé en ce que**
les phtalocyanines substituées contiennent des substituants attirant les électrons.

5. Procédé selon la revendication 4,
**caractérisé en ce que**
les substituants attirant les électrons sont des groupes halogène, nitro et/ou cyano.

6. Procédé selon l'une des revendications précédentes,
**caractérisé en ce qu'**
on utilise comme composé de bore l'acide ortho- ou méta-borique.

7. Procédé selon l'une des revendications précédentes,
**caractérisé en ce que**
la concentration du composé de bore est de 0,1 à 25 % en poids.

8. Procédé selon l'une des revendications précédentes,
**caractérisé en ce que**
la concentration du composé de bore est de 0,1 à 10 % en poids.

9. Procédé selon l'une des revendications précédentes,
**caractérisé en ce qu'**
on utilise des cycloalcanes comportant de 8 à 12 atomes de carbone comme composés de départ.

10. Procédé selon l'une des revendications précédentes,
**caractérisé en ce que**
la température de réaction est de 90 à 160°C.

11. Procédé selon l'une des revendications précédentes,
**caractérisé en ce que**
la température de la réaction est de 130 à 150°C.
